# EUROPÄISCHE PATENTSCHRIFT

(11) **EP 0 310 944 B1**
(45) Veröffentlichungstag und Bekanntmachung des Hinweises auf die Patenterteilung: **13.01.1993**
(21) Anmeldenummer: 88116094.9
(22) Anmeldetag: 29.09.1988
(51) Int. Cl.: C07C 315/00, C07C 321/12

(54) **Bis-oxethylsulfonylmethyl-aniline und Verfahren zu ihrer Herstellung**
Bisoxethylsulfonylmethyl anilines and process for their preparation
Anilines bis-oxéthylsulfonylméthyl et procédé de leur préparation

(30) Priorität: 03.10.1987 DE 3733504
(43) Veröffentlichungstag der Anmeldung: 12.04.1989
(73) Patentinhaber: HOECHST AKTIENGESELLSCHAFT, 65926 Frankfurt am Main (DE)
(72) Erfinder: Papenfuhs, Theodor, Dr., D-6000 Frankfurt am Main 50 (DE); Geisenberger, Josef, Dr., D-6233 Kelkheim (Taunus) (DE)

(56) Entgegenhaltungen:
- EP-A- 0 092 909
- EP-A- 0 166 155
- EP-A- 0 184 660
- GB-A- 931 595

## Beschreibung

Gegenstand der vorliegenden Erfindung sind neue Bis-oxethylsulfonylmethyl-aniline und Verfahren zu ihrer Herstellung. Die neuen Verbindungen sind wertvolle Vorprodukte zur Herstellung faserreaktiver Farbstoffe.

Die neuen Verbindungen der weiter unten angegebenen allgemeinen Formel (I) und deren Vorstufen der allgemeinen Formel (II) (siehe weiter unten) sind in der Literatur bisher nicht beschrieben. Aus der GB-PS 931 595 ist lediglich ein Derivat von (I) (4-Amino-2,6-bis-oxethylsulfonylmethyltoluol) bekannt, das als Vorprodukt für faserreaktive Farbstoffe dienen soll. Seine Herstellung erfolgt auf einem ökologisch ungünstigen Wege durch Chlormethylierung von 4-Nitrotoluol zu 4-Nitro-2,6-bis-chlormethyltoluol, das dann in an und für sich bekannter Weise durch Chlor-Austausch mit Mercaptoethanol, Oxidation mit Wasserstoffperoxid und Eisenreduktion zum Endprodukt umgesetzt wird.

Diese Synthesefolge verwendet als Schlüsselschritt die technisch problematische Chlormethylierung von Nitroaromaten mittels Formaldehyd/Salzsäure (unter Bildung von Dichlordimethylether, zu dessen Entsorgung hoher technischer Aufwand unabdingbar ist), erlaubt lediglich die Einführung von zur Nitrogruppe (und Aminogruppe) m-ständigen Reaktivsubstituenten und erfordert in der Chlor-Austauschstufe organische Lösungsmittel (vgl. Beispiel 4 der weiter obengenannten GB-PS).

Demgegenüber lassen sich die erfindungsgemäßen Verbindungen der allgemeinen Formel (I), in welcher R und X Wasserstoffatome bedeuten, die sich konstitutionell von der bekannten Verbindung dadurch unterscheiden, daß zumindest eine Reaktivgruppe nicht zum Nitro- (bzw. Amino-) substituenten m-ständig ist, aus den technisch durch Seitenkettenchlorierung der isomeren Xylole und anschließende destillative Reinigung leicht zugänglichen Bis-chlormethylbenzolen herstellen.

Charakteristisch für die Synthesefolge der vorliegenden Erfindung ist, daß die Stickstoff-Funktion erst nach dem Aufbau der Reaktiv-Gruppen in das Molekül eingeführt wird, woraus ein von Stand der Technik abweichendes Substitutionsmuster resultiert. Außerdem erlaubt die geänderte Reihenfolge der Verfahrensschritte, daß alle Stufen in Abwesenheit von organischen Lösungsmitteln durchführbar sind. Die neuen Verbindungen und Verfahren zu ihrer Herstellung gestatten daher eine breitere Variation der Substituenten zueinander und eine technisch mit gängigem Instrumentarium aus ökologisch einwandfrei synthetisierbaren Vorprodukten ohne Verwendung von Lösungsmitteln durchführbare Synthese.

Die vorliegende Erfindung betrifft somit neue Bis-oxethylsulfonylmethyl-aniline der allgemeinen Formel (I) 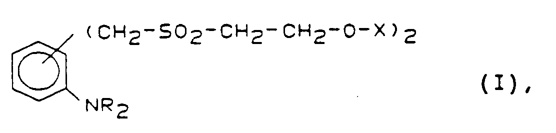
in welcher R und X Wasserstoffatome bedeuten, die beiden Seitenketten -CH₂-SO₂-CH₂-CH₂-O-X in ortho-, meta- oder para-Stellung zueinander stehen und die Gruppe -NR₂ in 4-Stellung im Fall der 1,2- oder 1,3-Stellung der beiden anderen Substituenten steht, und Verfahren zu ihrer Herstellung, indem man 1 Mol Xylylendichlorid (1,2-, 1,3- oder 1,4-Bis-chlormethylbenzol) mit mindestens 2 Mol Mercaptoethanol bei Temperaturen von 40 bis 150°C, vorzugsweise 70 bis 120°C, in wäßrigem Medium (in Abwesenheit von organischen Lösungsmitteln) in Gegenwart eines Säurebindemittels zum entsprechenden Bis-oxethylmercaptomethyl-benzol der allgemeinen Formel (II) 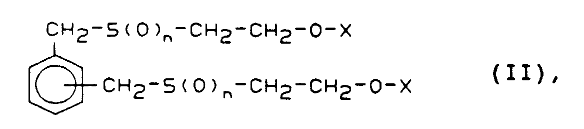
in welcher X ein Wasserstoffatom und n die Zahl 0 bedeutet, und die beiden Seitenketten in ortho-, meta- oder para-Position zueinanderstehen, umsetzt, dieses nach oder vorzugsweise ohne Zwischenisolierung mit mindestens 4 Mol, vorzugsweise 4,4 bis 5,6 Mol Wasserstoffperoxid bei Temperaturen von 50 bis 120°C, vorzugsweise 80 bis 100°C, bei einem pH-Wert von < 7, vorzugsweise < 4 in Gegenwart von Wolfram (VI)-Verbindungen als Katalysator zum entsprechenden Bis-oxethylsulfonylmethylbenzol der vorstehend genannten allgemeinen Formel (II), in welcher X ein Wasserstoffatom und n die Zahl 2 bedeuten, oxidiert, dieses mit mindestens 4 Mol, vorzugsweise 5 bis 7 Mol, wasserfreier Schwefelsäure bei Temperaturen von 30 bis 45°C in den entsprechenden Bis-Schwefelsäureester der genannten allgemeinen Formel (II), in welcher X die Gruppe -SO₃H und n die Zahl 2 bedeuten, überführt, diesen mit der mindestens stöchiometrischen Menge hochprozentiger Salpetersäure, ggf. im Gemisch mit wasserfreier Schwefelsäure, bei Temperaturen von 0 bis 60°C, vorzugsweise 25 bis 45°C, nitriert zum Bis-sulfatoethylsulfonylmethyl-nitrobenzol der Formel (III) 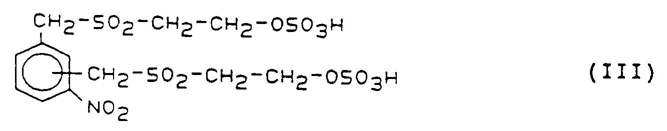
in welcher die beiden Seitenketten -CH₂-SO₂-CH₂-CH₂-OSO₃H in ortho-, meta- oder para-Stellung zueinander stehen, und die Nitrogruppe in 4-Stellung im Fall der 1,2- oder 1,3-Stellung der beiden genannten Seitenketten steht, dieses nach Zugabe von Wasser durch Erhitzen, zweckmäßigerweise bei Temperaturen von 100 bis 120°C, zum entsprechenden Bis-oxethylsulfonylmethyl-nitrobenzol verseift und letzteres nach Zwischenisolierung mit Eisen in waßrigem Medium oder vorzugsweise mit katalytisch aktiviertem Wasserstoff zum Bis-oxethylsulfonylmethyl-anilin der allgemeinen Formel (I), in welcher R und X Wasserstoffatome und n die Zahl 2 bedeuten, reduziert.

Zur Durchführung der einzelnen Stufen des erfindungsgemäßen Verfahrens sei im einzelnen folgendes ausgeführt:
1,2-, 1,3- oder 1,4-Bis-chlormethylbenzol (A) wird ohne Verwendung von organischen Lösungsmitteln in wäßrigem Medium mit Mercaptoethanol in Gegenwart eines Säurebinders zum entsprechenden Bis-oxethylmercaptomethylbenzol (B) umgesetzt: 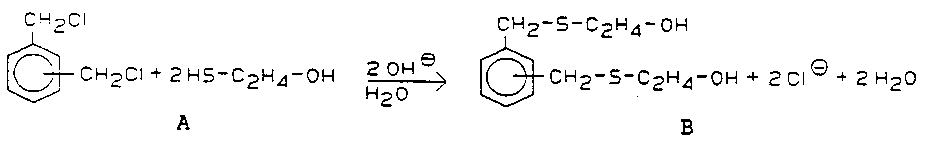
Zur quantitativen Umsetzung eines Mols Bis-chlormethylbenzol sind mindestens 2 Mol Mercaptoethanol nötig. Zur Erzielung hoher Ausbeuten in technisch vertretbarer Zeit hat sich ein molarer Mercaptoethanol-Überschuß von 10 bis 100 %, vorzugsweise 20 bis 50 %, als vorteilhaft erwiesen.

Als Säurebinder kommen die Oxide, Hydroxide und Carbonate der Alkali- und Erdalkalimetalle, vorzugsweise die Hydroxide und Carbonate der Alkalimetalle sowie die Oxide der Erdalkalimetalle, ganz besonders bevorzugt Kaliumhydroxid oder -carbonat und Magnesiumoxid in Frage. Sie werden in der Regel im Äquivalenzverhältnis 1 : 1 zum Mercaptoethanol eingesetzt. Geringe Überschüsse (bis etwa 30 %) an Säurebinder beeinflussen die Umsetzung nicht negativ und sind daher zulässig.

Die Kondensation wird bei Temperaturen von 40 bis 150°C, vorzugsweise 70 bis 120°C, ganz besonders bevorzugt bei 90 bis 100°C durchgeführt und ist in einigen Stunden beendet. Bei Temperaturen über 100°C muß im geschlossenen System unter Druck gearbeitet werden.

Die nach der Umsetzung sich als Feststoff oder Öl abscheidenden Bis-oxethylmercaptomethyl-benzole (B) werden gegebenenfalls durch Filtration oder Phasentrennung isoliert, können aber besonders vorteilhaft auch in der Kondensationsmischung weiterverarbeitet werden.

Die Oxidation der Verbindungen (B) zu den Bis-oxethylsulfonylmethylbenzolen (C) erfolgt mit Wasserstoffperoxid in Gegenwart von Wolfram (VI)-Katalysatoren (Natriumwolframat, Wolframtrioxid):

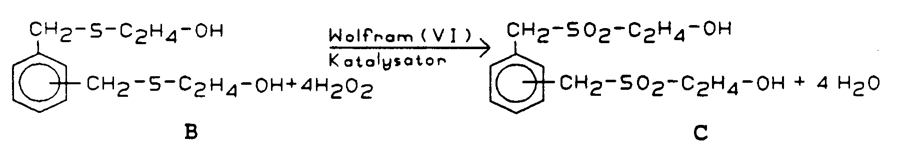

Dazu wird die isolierte Verbindung (B) in Wasser vorgelegt, oder, besonders vorteilhaft, direkt in der vorstehend genannten, angefallenen wäßrigen Kondensationsmischung bei einem pH-Wert < 7, vorzugsweise < 4, nach Zugabe des Katalysators (0,1 bis 10 Teile pro Mol Verbindung (B), vorzugsweise 1 bis 3 Teile) mit mindestens der 4-fachen Molmenge Wasserstoffperoxid, vorzugsweise der 4,4- bis 5,6-fachen Molmenge, bei Temperaturen von 50 bis 120°C, vorzugsweise 80 bis 100°C, versetzt und 2 bis 20 Stunden, vorzugsweise 4 bis 10 Stunden, nachgerührt. Die nach Abkühlen auf Raumtemperatur ausgefallenen Bis-oxethylsulfonylmethylbenzole (C) werden abgesaugt, gewaschen und getrocknet.

Zur Nitrierung der Verbindungen (C) müssen die Hydroxygruppen der Substituenten geschützt werden. Dies geschieht durch Veresterung mit Schwefelsäure zu den Halbestern der allgemeinen Formel (D):

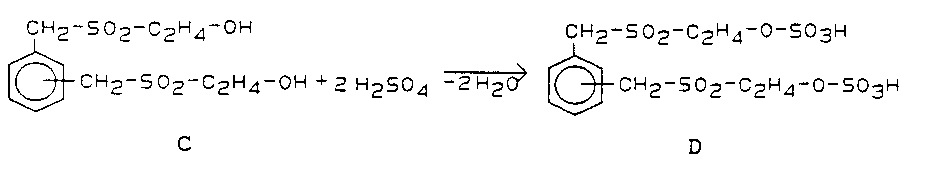

Zur vollständigen Veresterung ist ein Entfernen des Reaktionswassers erforderlich, was erfindungsgemäß durch Verwendung von wasserfreier Schwefelsäure im mindestens 4-molaren, vorteilhaft 5- bis 7-molaren Überschuß erreicht wird. Unter diesen Bedingungen ist eine quantitative Umsetzung bei mäßiger Temperatur ( 30 bis 45°C) in wenigen Stunden möglich.

Die resultierende schwefelsaure Lösung der Verbindungen der Formel (D) dient erfindungsgemäß direkt als Nitriermedium. Eine Isolierung der Bis-sulfatoethylsulfonylmethyl-benzole (D) bietet keine Vorteile. Die Nitrierung erfolgt durch Zugabe der mindestens stöchiometrischen Menge hochprozentiger Salpetersäure, gegebenenfalls im Gemisch mit wasserfreier Schwefelsäure (allgemein als "Misch- oder Nitriersäuren" bekannt) im Verlaufe von 2 bis 10, vorzugsweise 3 bis 6 Stunden bei Temperaturen von 0 bis 60°C, vorzugsweise bei 45°C. Zur Vervollständigung der Nitrierung kann es von Vorteil sein, die Salpeter- oder Nitriersäure in geringem Überschuß (5 bis 20 Molprozent) anzuwenden:

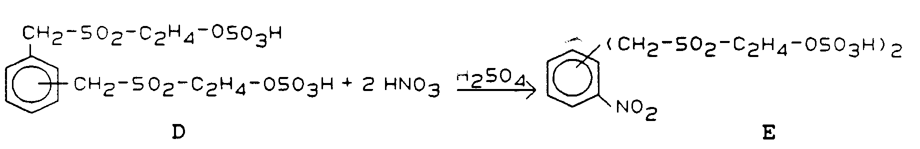

Das nach beendeter Nitrierung in schwefelsaurer Lösung vorliegende Bis-ß-sulfatoethylsulfonylmethyl-nitrobenzol der allgemeinen Formel E bzw. I (R = O, X = -SO₃H) wird erfindungsgemäß in der Regel ebenfalls nicht isoliert (falls erwünscht, kann es durch Aussalzen mit beispielsweise Natriumsulfat gefällt und durch Filtration von der Hauptmenge Schwefelsäure abgetrennt werden), sondern nach Zugabe von Wasser (ein- bis sechsfache Gewichtsmenge, vorzugsweise drei- bis vierfache Gewichtsmenge, bezogen auf den Gesamteinsatz Schwefelsäure), durch mehrstündiges Erhitzen am Rückfluß (3 bis 10 Stunden, vorzugsweise 4 bis 6 Stunden bei 100 bis 120°C) zur freien Oxethylsulfonylmethylverbindung der allgemeinen Formel (F) verseift:

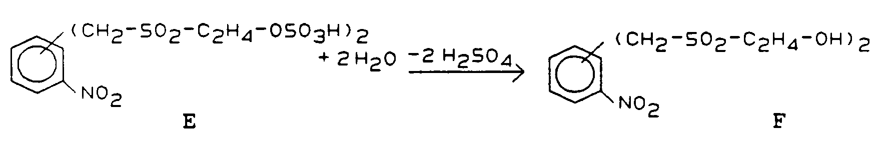

Beim Abkühlen auf Temperaturen von -5°C bis +10°C fällt das Bis-oxethylsulfonylmethyl-nitrobenzol (F) aus. Es wird abgesaugt, gewaschen und gegebenenfalls getrocknet.

Zur abschließenden Reduktion der Nitrogruppe kann sowohl eine wäßrige Reduktion mit Eisen (Béchamp-Reduktion), oder, besonders vorteilhaft, eine wäßrige Hydrierung mit katalytisch aktiviertem Wasserstoff angewandt werden. Als Katalysatoren kommen sowohl handelsübliche Nickel-Katalysatoren als auch käufliche Edelmetall-Kontakte, wie beispielsweise Platin oder Palladium auf inerten Trägern, vorzugsweise auf Aktivkohlen hoher spezifischer Oberfläche, in Betracht.

Die Reduktion verläuft bei erhöhten Temperaturen (70 bis 120°C, vorzugsweise 80 bis 100°C) in wäßriger Lösung oder Suspension in wenigen Stunden (in 1 bis 5 Stunden, vorzugsweise im erhöhten Temperaturbereich in 1,5 bis 3 Stunden) quantitativ und liefert bei Reduktionstemperatur wäßrige Lösungen der gewünschten Bis-oxethylsulfonylmethyl-anilin-Verbindungen der allgemeinen Formel G bzw. I (mit R und X = Wasserstoff), aus denen die suspendierten Eisenoxidhydrate (im Falle der Béchamp-Reduktion) bzw. die Hydrierkatalysatoren durch Klärfiltration vorteilhaft abtrennbar sind:

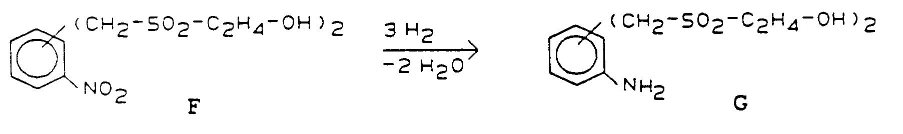

Aus dem Klärfiltrat können die Bis-oxethylsulfonylmethyl-anilin-Verbindungen, gegebenenfalls nach Konzentrierung durch Abdestillieren von Wasser im Vakuum, durch Kühlen auf Temperaturen von -5 bis +20°C und/oder Aussalzen mit beispielsweise Natriumchlorid oder -sulfat ausgefällt und durch Filtration oder Zentrifugieren isoliert werden.

Die Ausbeuten und Selektivitäten sind in allen Stufen überraschend hoch und erreichen in den meisten Fällen nahezu theoretische Werte.

Die neuen Bis-oxethylsulfonylmethyl-aniline der allgemeinen Formel I (mit R und X = H) eignen sich hervorragend als Diazokomponenten für faserreaktive Azofarbstoffe mit besonders hohem Fixiergrad und erlauben dadurch einen erheblichen technischen Fortschritt in dieser bedeutenden Farbstoffklasse.

Die nachfolgenden Beispiele sollen die Erfindung näher erläutern, ohne sie darauf zu beschränken. Die angegebenen Teile sind, soweit nicht anders angegeben, Gewichtsteile.

### Beispiel 1

Eine gerührte Mischung aus 1000 Teilen Wasser, 350 Teilen 1,4-Bis-chlormethylbenzol (p-Xylylendichlorid), 437 Teilen Mercaptoethanol und 392 Teilen Kaliumcarbonat wird 3 Stunden auf 90 bis 95°C erhitzt. Nach dieser Zeit erhält man ein einheitliches HPLC-Chromatogramm, Ausgangsprodukt ist nicht mehr nachweisbar. Man kühlt auf 10°C ab, saugt den ausgefallenen Niederschlag ab, wäscht mit Eiswasser neutral und trocknet im Vakuum bei 60°C.

Man erhält 512 Teile 1,4-Bis-oxethylmercaptomethyl-benzol der Formel 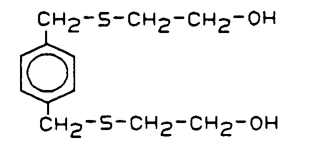
vom Schmelzpunkt 90 bis 92°C.

| | |
|---|---|
| Analyse: | S: 24,9/24,8 % (ber. 24,8 %) |
| | Cl: < 0,03 % (ber. 0,0 %) |

### Beispiel 2

Eine Mischung aus 700 Teilen 1,2-Bis-chlormethylbenzol (o-Xylylendichlorid), 2000 Teilen Wasser und 756 Teilen Mercaptoethanol wird bei 60°C in 15 Minuten unter Rühren mit 200 Teilen Magnesiumoxid versetzt, wobei leichte Kühlung zum Halten der Temperatur erforderlich ist. Anschließend heizt man 3 Stunden auf 95 bis 100°C, wonach ein HPLC-Chromatogramm vollständige Umsetzung anzeigt. Man kühlt auf 15°C, trennt das abgeschiedene Öl ab, wäscht dieses durch Ausrühren mit ionenfreiem Wasser und erneute Phasentrennung und trocknet die organische Phase durch Abdestillieren im Vakuum von 2664,4 Pascal (20 Torr), bis keine Brüden mehr übergehen.

Man erhält 1030 Teile 1,2-Bis-oxethylmercaptomethyl-benzol der Formel 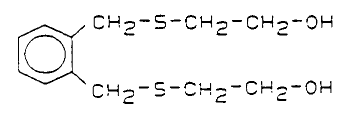
als farblose Flüssigkeit, die nicht unzersetzt destillierbar ist.

| | |
|---|---|
| Analyse: | S: 24,95/24,85 % (ber. 24,8 %) |
| | Cl: < 0,03 % (ber. 0,0 %) |

### Beispiel 3

Ersetzt man im vorstehenden Beispiel 2 das 1,2-Bis-chlormethylbenzol durch 1,3-Bis-chlormethylbenzol (m-Xylylendichlorid) und arbeitet sonst in der gleichen Weise, so erhält man in vergleichbarer Ausbeute das 1,3-Bis-oxethylmercaptomethyl-benzol der Formel 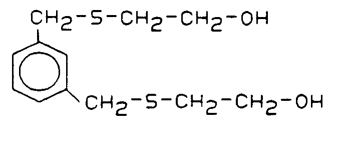
als farbloses, nicht destillierbares Öl.

### Beispiel 4

Zu einer gerührten Vorlage aus 103,2 Teilen 1,4-Bis-oxethylmercaptomethylbenzol (hergestellt nach Beispiel 1), 20 Teilen Eisessig und 1,6 Teilen Natriumwolframat tropft man bei 90°C innerhalb 1 Stunden 168 Teile 35 %iges wäßriges Wasserstoffperoxid, rührt 3 Stunden bei 90 bis 95°C nach (ein HPLC-Chromatogramm zeigt vollständige Oxidation), kühlt auf 20°C ab, isoliert den ausgefallenen Niederschlag durch Absaugen, wäscht mit Eiswasser neutral und trocknet im Vakuum bei 80°C.

Man erhält 125,0 Teile 1,4-Bis-oxethylsulfonylmethyl-benzol der Formel 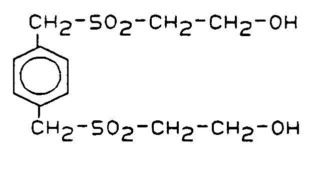
in Form farbloser Kristalle vom Schmelzpunkt 213 bis 215°C. Der Reingehalt (HPLC) beträgt 98,7 %.

### Beispiel 5

Ersetzt man im vorgehenden Beispiel 4 das 1,4-Bis-oxethylmercaptomethyl-benzol durch die gleiche Menge des 1,3-Isomeren und arbeitet im übrigen in der angegebenen Weise, so erhält man 105,1 Teile 1,3-Bis-oxethylsulfonylmethyl-benzol der Formel 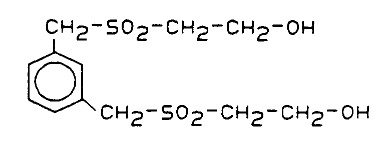
als farbloses, bei 111 bis 112°C schmelzendes Kristallisat mit einem Reingehalt (HPLC) von 99,1 %.

### Beispiel 6

Eine Mischung aus 258 Teilen 1,2-Bis-oxethylmercaptomethyl-benzol, 250 Teilen 10 %iger Schwefelsäure und 4 Teilen Natriumwolframat wird, bei 60°C beginnend, so schnell mit 420 Teilen 35 %igem wäßrigem Wasserstoffperoxid versetzt, daß die Innentemperatur 95°C nicht überschreitet (ca. 70 Minuten). Anschließend wird 5 Stunden bei 95°C nachgerührt (Kontroll-HPLC auf vollständige Umsetzung), auf 10°C abgekühlt und der ausgefallene Niederschlag durch Filtration isoliert. Nach Waschen mit Eiswasser und Trocknen im Vakuum bei 80°C erhält man 304 Teile 1,2-Bis-oxethylsulfonylmethyl-benzol der Formel 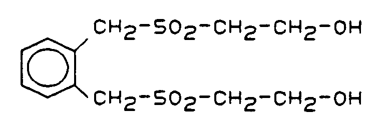
in Form farbloser, bei 116 bis 118°C schmelzende Kristalle. Der Reingehalt (HPLC) beträgt 98,4 %.

### Beispiel 7 (Eintopfverfahren)

700 Teile 1,4-Bis-chlormethylbenzol (p-Xylylendichlorid), 750 Teile Mercaptoethanol und 1500 Teile Wasser werden unter Rühren gemischt und auf 50 bis 55°C erwärmt. Dann trägt man in 30 Minuten 200 Teile Magnesiumoxid so ein, daß die Innentemperatur nicht über 60°C steigt, heizt anschließend 3 Stunden auf 90 bis 95°C (laut HPLC-Chromatogramm ist die Ausgangsverbindung vollständig umgesetzt), stellt mit 2n Schwefelsäure auf pH 7,0 und kühlt auf 60°C ab. Darauf setzt man nacheinander 150 Teile Eisessig und 8 Teile Wolframtrioxid zu und tropft dann in 60 Minuten gleichmäßig 1250 Teile Wasserstoffperoxid 35 %ig zu. Dabei darf die Innentemperatur auf 90°C ansteigen. Man rührt ca. 5 Stunden bei 90 bis 95°C nach, bis eine Probe im HPLC-Chromatogramm einheitliches Zielprodukt anzeigt, kühlt auf 20°C ab und isoliert den ausgefallenen Niederschlag durch Filtration. Nach Waschen mit Eiswasser und Trocknen im Vakuum bei 100°C erhält man 842 Teile 1,4-Bis-oxethylsulfonylmethyl-benzol der Formel 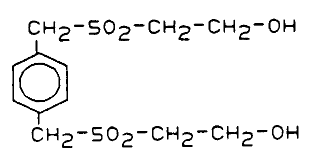
in Form leicht gelblicher Kristalle vom Schmelzpunkt 210 bis 212°C und einem Reingehalt (HPLC) von 96,9 %.

Ersetzt man das 1,4-Bis-chlormethylbenzol durch 1,2- oder 1,3-Bis-chlormethylbenzol und arbeitet im übrigen in der angegebenen Weise, so erhält man das 1,2- bzw. 1,3-Bis-oxethylsulfonylmethyl-benzol in vergleichbarer Ausbeute und Qualität.

### Beispiel 8

Man trägt unter Rühren 80,5 Teile 1,4-Bis-oxethylsulfonylmethyl-benzol in 612,5 Teile 100 %ige Schwefelsäure ein und erhitzt solange auf 35 bis 40°C, bis eine Probe im HPLC-Chromatogramm quantitative Veresterung anzeigt (ca. 4 bis 5 Stunden). Anschließend tropft man innerhalb 4 Stunden bei 30 bis 40°C 57 Teile einer Mischung aus 30 Gewichtsteilen 100 %iger Salpetersäure und 70 Gewichtsteilen 100 %iger Schwefelsäure (sog. "M3-Säure") zu, rührt 2 Stunden bei 45°C bis zur vollständigen Nitrierung nach (Kontrolle durch HPLC-Chromatogramm) und zersetzt dann die Reaktionsmischung durch Eingießen in 2500 Teile Kaltwasser. Die resultierende wäßrige-saure Lösung wird zur Verseifung der Schwefelsäureester-Gruppen 5 Stunden am Rückfluß erhitzt (ca. 110°C), dann mit 5 Teilen Aktivkohle versetzt, geklärt und das Klärfiltrat auf 0 bis 5°C kaltgerührt. Der dabei ausfallende Niederschlag wird abgesaugt, neutral gewaschen und im Vakuum bei 80°C getrocknet. Man erhält 89,0 Teile 2,5-Bis-oxethylsulfonylmethyl-nitrobenzol der Formel 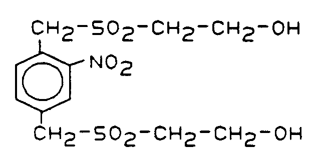
in Form farbloser Kristalle vom Schmelzpunkt 125 bis 126,5°C, deren Reingehalt (HPLC) 98,8 % beträgt.

Ersetzt man die "M3-Säure" durch 17,5 Teile 98 %ige Salpetersäure und arbeitet im übrigen in der angegebenen Weise, so erhält man ein vergleichbares Ergebnis.

### Beispiel 9

Arbeitet man analog Beispiel 8, ersetzt aber das 1,4-Bis-oxethylsulfonylmethyl-benzol durch das 1,3-Isomere, so erhält man 82,4 Teile 2,4-Bis-oxethylsulfonylmethyl-nitrobenzol der Formel 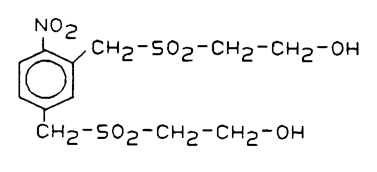
als leicht gelb gefärbtes Kristallisat vom Schmelzpunkt 211 bis 212°C mit einem Reingehalt (HPLC) von 97,9 %.

### Beispiel 10

Arbeitet man analog Beispiel 8, ersetzt aber das 1,4-Bis-oxethylsulfonylmethyl-benzol durch das 1,2-Isomere, so erhält man 85,4 Teile 3,4-Bis-oxethylsulfonylmethyl-nitrobenzol der Formel 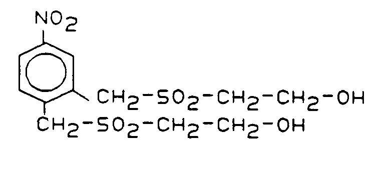
als farbloses Kristallisat vom Schmelzpunkt 126 bis 128°C mit einem Reingehalt (HPLC) von 98,2 %.

### Beispiel 11

Zu einer auf 80 bis 85°C erwärmten Mischung aus 40 Teilen Eisenpulver und 200 Teilen Wasser gibt man innerhalb 30 Minuten unter Rühren gleichmäßig 100 Teile 2,4-Bis-oxethylsulfonylmethyl-nitrobenzol und hält während des Eintragens die Reaktionstemperatur auf 80 bis 85°C. Nach beendeter Zugabe rührt man 30 Minuten nach, stellt mit wäßriger Sodalösung einen pH-Wert von 8,5 ein und klärt heiß vom ausgefallenen Eisenhydroxid. Der Filterrückstand wird zweimal mit wenig heißem Wasser gewaschen. Anschließend werden die vereinigten filtrate im Vakuum auf 150 Vol.-Teile eingeengt und nachfolgend auf 0 bis 5°C kaltgerührt. Der ausgefallene schwach bräunlich gefärbte Niederschlag wird auf einer Nutsche isoliert, mit wenig Eiswasser abgedeckt und bei 60°C im Vakuum getrocknet. Man erhält 84,7 Teile 2,4-Bis-oxethylsulfonylmethyl-anilin der Formel 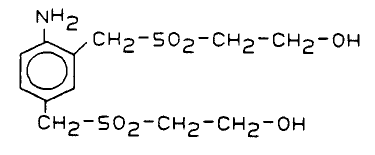
vom Schmelzpunkt 183 bis 185°C und einem Reingehalt (durch Diazotierung) von 99,5 %.

| | | |
|---|---|---|
| Analyse: | C: 42,6 % (ber. 42,72 %), | H: 5,85 % (ber. 5,68 %), |
| | N: 4,05 % (ber. 4,15 %), | S: 18,95 % (ber. 19,00 %). |

### Beispiel 12

Ersetzt man im Beispiel 11 das 2,4-Bis-oxethylsulfonylmethyl-nitrobenzol durch das 2,5-Isomere und arbeitet im übrigen in der angegebenen Weise, so erhält man das 2,5-Bis-oxethylsulfonylmethyl-anilin der Formel 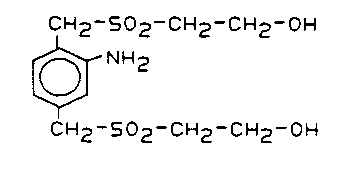
als beigegefärbtes Kristallisat vom Schmelzpunkt 196 bis 198°C in vergleichbarer Ausbeute und Qualität. Durch Elementaranalyse wird die vorstehend angegebene Formel belegt.

### Beispiel 13

In einem Hydrierautoklav werden 3000 Teile Wasser und 150 Teile 2,5-Bis-oxethylsulfonylmethyl-nitrobenzol vorgelegt und 10 Teile Edelmetallkatalysator (5 % Palladium auf Kohle) zugegeben. Der Autoklav wird verschlossen und der Gasraum durch dreimaliges Spülen zuerst mit Stickstoff, dann mit Wasserstoff sauerstoff- und stickstofffrei gemacht.

Anschließend werden 40 bar Wasserstoff aufgedrückt und auf 90°C aufgeheizt. Der Wasserstoffdruck wird durch stetiges Nachdrücken von Wasserstoff auf 40 bis 45 bar gehalten. Nach 2 Stunden bei 90°C bricht die Wasserstoffaufnahme ab. Der Autoklav-Inhalt wird heiß über ein Druckfilter vom Katalysator geklärt und das Filtrat auf 0 bis 5°C kaltgerührt. Die ausgefallenen farblosen Kristalle werden abgesaugt, mit wenig Eiswasser gewaschen und im Vakuum bei 60°C getrocknet. Man erhält 110 Teile 2,5-Bis-oxethylsulfonylmethyl-anilin der Formel 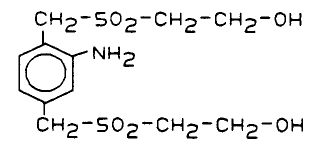
vom Schmelzpunkt 198 bis 199°C mit einem Reingehalt von 99,9 % (bestimmt durch Diazotierung).

### Beispiel 14

Beispiel 13 wird exakt wiederholt, lediglich mit dem Unterschied, daß an Stelle der 3000 Teile Wasser die gemäß Beispiel 13 angefallene wäßrige Mutterlauge (ca. 3050 Teile), und an Stelle von Frischkontakt der aus der Hydrierung durch Klärfiltration abgetrennte Palladium-Katalysator eingesetzt wird.

Man erhält 122 Teile 2,5-Bis-oxethylsulfonylmethyl-anilin vom Schmelzpunkt 198 bis 199°C und einem Reingehalt (Diazotierung) von 99,8 %.

### Beispiele 15 bis 23

Verfährt man weiter, wie in den Beispielen 13 und 14 beschrieben und setzt jeweils 150 Teile 2,5-Bis-oxethylsulfonylmethyl-nitrobenzol ein, dazu jeweils die wäßrige Mutterlauge und den Palladium-Kontakt des vorhergehenden Ansatzes, so erhält man jeweils ca. 128 Teile 2,5-Bis-oxethylsulfonylmethyl-anilin vom Schmelzpunkt 197 bis 199°C und einem Reingehalt (Diazotierung) von > 99 %, d.h., Mutterlauge und Katalysator sind jeweils mindestens 10 mal ohne Produktverschlechterung oder Ausbeutesenkung rückführbar.

### Beispiel 24

Ersetzt man in Beispiel 13 den Palladium-Katalystor durch einen handelsüblichen Platin- oder Nickel-Trägerkatalysator und arbeitet im übrigen in der dort angegebenen Weise, so erhält man das 2,5-Bis-oxethylsulfonylmethyl-anilin in vergleichbarer Ausbeute und Qualität.

### Beispiel 25

Ersetzt man im Beispiel 13 das 2,5-Bis-oxethylsulfonyl-nitrobenzol durch das 3,4-Isomere (vgl. Beispiel 10) und arbeitet im übrigen nach den dortigen Angaben, so erhält man das 3,4-Bis-oxethylsulfonylmethyl-anilin der Formel 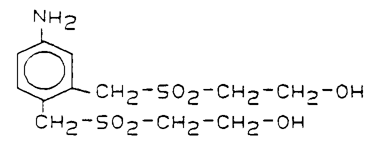
in Form farbloser Kristalle vom Schmelzpunkt 168 bis 171°C mit einem Reingehalt (Diazotierung) von 99,2 %.

## Patentansprüche

1. Bis-oxethylsulfonylmethyl-aniline der Formel 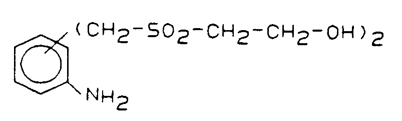 in welcher die beiden Seitenketten -CH₂-SO₂-CH₂-CH₂-OH in ortho-, meta- oder para-Stellung zueinander stehen, und die Aminogruppe in 4-Stellung im Fall der 1,2- oder 1,3-Stellung der beiden genannten Seitenketten steht.

2. Bis-oxethylsulfonylmethyl-nitrobenzole der Formel 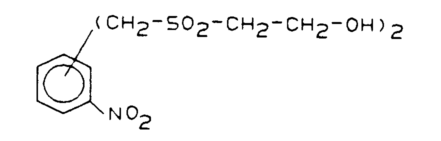 in welcher die beiden Seitenketten -CH₂-SO₂-CH₂-CH₂-OH in ortho-, meta- oder para-Stellung zueinander stehen, und die Nitrogruppe in 4-Stellung im Fall der 1,2- oder 1,3-Stellung der beiden genannten Seitenketten steht.

3. Bis-oxethylsulfonylmethyl-benzole der Formel 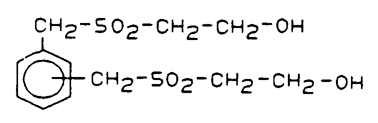 in welcher die beiden Seitenketten in ortho-, meta- oder para-Stellung zueinander stehen.

4. Bis-oxethylmercaptomethyl-benzole der Formel 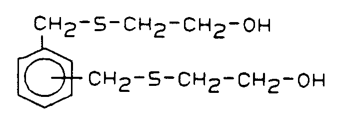 in welcher die beiden Seitenketten in ortho-, meta- oder para-Stellung zueinander stehen.

5. Verfahren zur Herstellung von Bis-oxethylsulfonylmethyl-anilinen der Formel 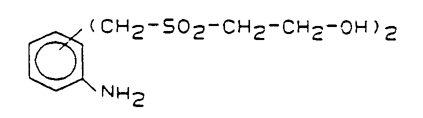 in welcher die beiden Seitenketten -CH₂-SO₂-CH₂-CH₂-OH in ortho-, meta- oder para-Stellung zueinander stehen, und die Aminogruppe in 4-Stellung im Fall der 1,2- oder 1,3-Stellung der beiden genannten Seitenketten steht, dadurch gekennzeichnet, daß man 1 Mol Xylylendichlorid (1,2-, 1,3 oder 1,4-Bis-chlormethylbenzol) mit mindestens 2 Mol Mercaptoethanol bei Temperaturen von 40° bis 150°C in wäßrigem Medium in Gegenwart eines Säurebindemittels zum entsprechenden Bis-oxethylmercaptomethyl-benzol der Formel 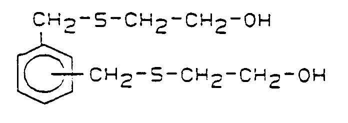 in welcher die beiden Seitenketten in ortho-, meta- oder para-Stellung zueinander stehen, umsetzt, dieses mit mindestens 4 Mol Wasserstoffperoxid bei Temperaturen von 50 bis 120°C bei einem pH-Wert < 7 in Gegenwart von Wolfram (VI)-Verbindungen als Katalysator zum entsprechenden Bis-oxethylsulfonylmethyl-benzol der Formel 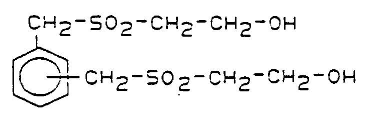 in welcher die beiden Seitenketten in ortho-, meta- oder para-Stellung zueinanderstehen, oxidiert, dieses mit mindestens 4 Mol wasserfreier Schwefelsäure bei Temperaturen von 30 bis 45°C in den entsprechenden Bis-Schwefelsäureester überführt, letzteren mit der mindestens stöchiometrischen Menge hochprozentiger Salpetersäure bei Temperaturen von 0 bis 60°C nitriert zum Bis-sulfatoethylsulfonylmethyl-nitrobenzol der Formel 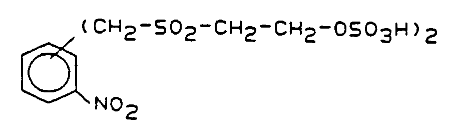 in welcher die beiden Seitenketten -CH₂-SO₂-CH₂-CH₂-OSO₃H in ortho-, meta- oder para-Stellung zueinander stehen, und die Nitrogruppe in 4-Stellung im Fall der 1,2- oder 1,3-Stellung der beiden genannten Seitenketten steht, dieses nach Zugabe von Wasser durch Erhitzen zum entsprechenden Bis-oxethylsulfonylmethyl-nitrobenzol verseift und letzteres nach Zwischenisolierung zum entsprechenden Bis-oxethylsulfonylmethyl-anilin reduziert.

6. Verfahren nach Anspruch 5, dadurch gekennzeichnet, daß man als Säurebinder die Oxide, Hydroxide oder Carbonate der Alkali- oder Erdalkalimetalle verwendet.

7. Verfahren nach mindestens einem der Ansprüche 5 und 6, dadurch gekennzeichnet, daß man die Oxidation in Gegenwart von Natriumwolframat oder Wolframtrioxid durchführt.

8. Verfahren nach mindestens einem der Ansprüche 5 bis 7, dadurch gekennzeichnet, daß man die Reduktion mit Eisen in wäßrigem Medium durchführt.

9. Verfahren nach mindestens einem der Ansprüche 5 bis 7, dadurch gekennzeichnet, daß man die Reduktion in wäßrigem Medium mit katalytisch aktiviertem Wasserstoff durchführt.

10. Verfahren nach mindestens einem der Ansprüche 5 bis 9, dadurch gekennzeichnet, daß man die Verseifung durch Erhitzen am Rückfluß auf Temperaturen von 100 bis 120°C durchführt.

## Claims

1. A bis(hydroxyethylsulfonylmethyl)aniline of the formula 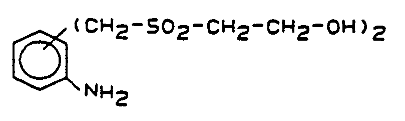 in which the two side chains -CH₂-SO₂-CH₂-CH₂-OH are in the ortho-, meta- or para-position relative to one another and the amino group is in the 4-position in the case where the two side chains mentioned are in the 1,2-or 1,3-position.

2. A bis(hydroxyethylsulfonylmethyl)nitrobenzene of the formula 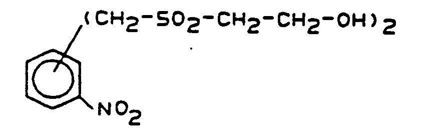 in which the two side chains -CH₂-SO₂-CH₂-CH₂-OH are in the ortho-, meta- or para-position relative to one another and the nitro group is in the 4-position in the case where the two side chains mentioned are in the 1,2-or 1,3-position.

3. A bis(hydroxyethylsulfonylmethyl)benzene of the formula 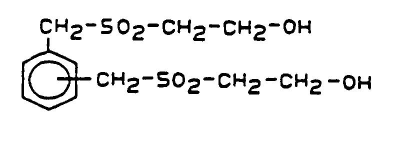 in which the two side chains are in the ortho-, meta- or para-position relative to one another.

4. A bis(hydroxyethylmercaptomethyl)benzene of the formula 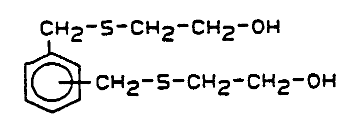 in which the two side chains are in the ortho-, meta- or para-position relative to one another.

5. A process for the preparation of bis(hydroxyethylsulfonylmethyl)anilines of the formula 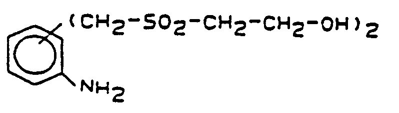 in which the two side chains -CH₂-SO₂-CH₂-CH₂-OH are in the ortho-, meta- or para-position relative to one another and the amino group is in the 4-position in the case where the two side chains mentioned are in the 1,2-or 1,3-position, which comprises reacting 1 mol of xylylene dichloride (1,2-, 1,3-, or 1,4-bis(chloromethyl)benzene) with at least 2 mol of mercaptoethanol at temperatures from 40° to 150°C in an aqueous medium in the presence of an acid-binding agent to give the corresponding bis(hydroxyethylmercaptomethyl)benzene of the formula 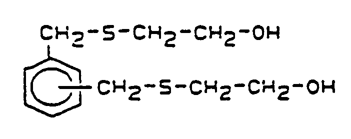 in which the two side chains are in the ortho-, meta- or para-position relative to one another, oxidizing this product with at least 4 mol of hydrogen peroxide at temperatures from 50 to 120°C at a pH < 7 in the presence of a tungsten (VI) compound as catalyst to give the corresponding bis(hydroxyethylsulfonylmethyl)benzene of the formula 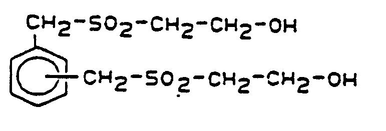 in which the two side chains are in the ortho-, meta- or para-position relative to one another, converting this product with at least 4 mol of anhydrous sulfuric acid at temperatures of 30 to 45°C to the corresponding bis(sulfuric ester), nitrating the latter with at least the stoichiometric amount of high-percentage nitric acid at temperatures from 0 to 60°C to give the bis(sulfatoethylsulfonylmethyl)nitrobenzene of the formula 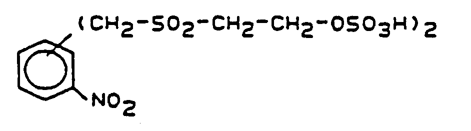 in which the two side chains -CH₂-SO₂-CH₂-CH₂-OSO₃H are in the ortho-, meta- or para-position relative to one another and the nitro group is in the 4-position in the case where the two side chains mentioned are in the 1,2- or 1,3-position, hydrolyzing this product after the addition of water by heating to give the corresponding bis(hydroxyethylsulfonylmethyl)nitrobenzene and reducing the latter, after isolation of the intermediate, to the corresponding bis(hydroxyethylsulfonylmethyl)aniline.

6. The process as claimed in claim 5, wherein the acid-binding agents used are the oxides, hydroxides or carbonates of the alkali metals or alkaline earth metals.

7. The process as claimed in at least one of claims 5 and 6, wherein the oxidation is carried out in the presence of sodium tungstate or tungsten trioxide.

8. The process as claimed in one of claims 5 to 7, wherein the reduction with iron is carried out in an aqueous medium.

9. The process as claimed in at least one of claims 5 and 7, wherein the reduction is carried out in an aqueous medium with catalytically activated hydrogen.

10. The process as claimed in at least one of claims 5 to 9, wherein the hydrolysis is carried out by refluxing to temperatures from 100 to 120°C.

## Revendications

1. Bis-(hydroxyéthyl-sulfonyl-méthyl)-anilines répondant à la formule : 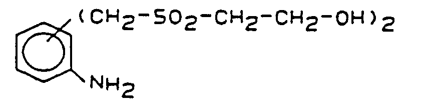 dans laquelle les deux chaînes latérales -CH₂-SO₂-CH₂-CH₂-OH sont en position ortho, méta ou para l'une par rapport à l'autre, et le radical amino se trouve à la position 4 lorsque les deux chaînes latérales en question sont en 1,2 ou en 1,3.

2. Bis-(hydroxyéthyl-sulfonyl-méthyl)-nitrobenzènes répondant à la formule : 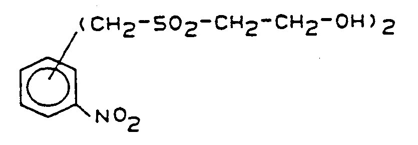 dans laquelle les deux chaînes latérales -CH₂-SO₂-CH₂-CH₂-OH sont en position ortho, méta ou para l'une par rapport à l'autre, et le radical nitro se trouve à la position 4 lorsque les deux chaînes latérales en question sont en 1,2 ou en 1,3.

3. Bis-(hydroxyéthyl-sulfonyl-méthyl)-benzènes répondant à la formule : 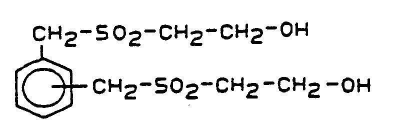 dans laquelle les deux chaînes latérales sont en position ortho, méta ou para l'une par rapport à l'autre.

4. Bis-(hydroxyéthyl-thio-méthyl)-benzènes répondant à la formule : 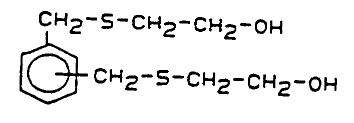 dans laquelle les deux chaînes latérales sont en position ortho, méta ou para l'une par rapport à l'autre.

5. Procédé pour préparer des bis-(hydroxyéthyl-sulfonyl-méthyl)-anilines répondant à la formule : 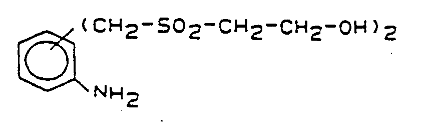 dans laquelle les deux chaînes latérales -CH₂-SO₂-CH₂-CH₂-OH sont en position ortho, méta ou para l'une par rapport à l'autre, et le radical amino occupe la position 4 lorsque les deux chaînes latérales citées sont en 1,2 ou en 1,3,
procédé caractérisé en ce qu'on fait réagir 1 mol de dichlorure de xylylène (1,2-, 1,3- ou 1,4-bis-chlorométhyl-benzène) avec au moins 2 mol de mercapto-éthanol, à des températures de 40 et 150°C, en milieu aqueux, en présence d'un accepteur d'acides, pour obtenir le bis-(hydroxyéthyl-thio-méthyl)-benzène répondant à la formule générale : 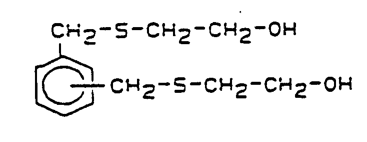 dans laquelle les deux chaînes latérales sont en position ortho, méta ou para l'une par rapport à
l'autre, on oxyde celui-ci avec au moins 4 mol de peroxyde d'hydrogène, à des températures de 50 et 120°C, à un pH inférieur à 7, en présence de composés du tungstène(VI) comme catalyseur, oxydation qui conduit au bis-(hydroxy-éthyl-sulfonyl-méthyl)-benzène correspondant qui répond à la formule : 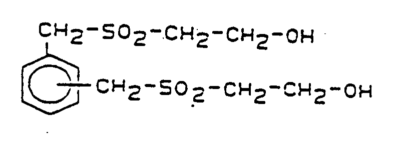 dans laquelle les deux chaînes latérales sont en position ortho, méta ou para l'une par rapport à l'autre,
on transforme celui-ci, avec au moins 4 mol d'acide sulfurique anhydre, à des températures de 30 et 45°C, en le bis-ester sulfurique correspondant, on nitre ce dernier avec une quantité d'acide nitrique très concentré au moins égale à la quantité stoechiométrique, à des températures de 0 et 60°C, de manière à obtenir le bis-(sulfatoéthyl-sulfonyl-méthyl)-nitrobenzène répondant à la formule : 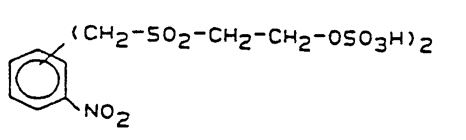 dans laquelle les deux chaînes latérales -CH₂-SO₂-CH₂-CH₂-OSO₃H sont en position ortho, méta ou para l'une par rapport à l'autre, et le radical nitro est à la position 4 lorsque les deux chaînes latérales mentionnées sont en 1,2 ou en 1,3,
ensuite, après y avoir ajouté de l'eau, on saponifie ce composé par chauffage pour le convertir en le bis-(hydroxyéthyl-sulfonyl-méthyl)-nitrobenzène correspondant, et on réduit ce dernier, après l'avoir isolé intermédiairement, pour obtenir la bis-(hydroxyéthyl-sulfonyl-méthyl)-aniline correspondante.

6. Procédé selon la revendication 5 caractérisé en ce qu'on utilise, comme accepteurs d'acides, les oxydes, hydroxydes ou carbonates des métaux alcalins ou des métaux alcalino-terreux.

7. Procédé selon au moins une des revendications 5 et 6, caractérisé en ce qu'on effectue l'oxydation en présence de tungstate de sodium ou de trioxyde de tungstène.

8. Procédé selon au moins une des revendications 5 à 7, caractérisé en ce qu'on effectue la réduction au moyen de fer en milieu aqueux.

9. Procédé selon au moins une des revendications 5 à 7, caractérisé en ce qu'on effectue la réduction en milieu aqueux avec de l'hydrogène catalytiquement activé.

10. Procédé selon au moins une des revendications 5 à 9, caractérisé en ce qu'on effectue la saponification par chauffage à reflux à des températures de 100 à 120°C.
